# EUROPEAN PATENT APPLICATION

(11) **EP 3 722 070 A1**
(43) Date of publication of application: **14.10.2020**
(21) Application number: 18886693.3
(22) Date of filing: 05.12.2018
(51) Int. Cl.: B29C 59/02, A61M 37/00, B23K 26/067, B23K 26/352, B29C 59/16

(54) **FUNCTIONAL MEMBER AND METHOD FOR MANUFACTURING SAME**

(30) Priority: 05.12.2017 JP 2017233102
(71) Applicant: Furukawa Electric Co., Ltd., Chiyoda-ku Tokyo 100-8322 (JP)
(72) Inventor: IWAMA, Masaki, Tokyo 100-8322 (JP)
(74) Representative: J A Kemp LLP
(86) International application number: PCT/JP2018/044762
(87) International publication number: WO 2019/111959

(57) **Abstract**

A functional member includes a plurality of microneedles on a surface. Adjacent microneedles are spaced apart by a pitch ranging from 1 to 500 µm, and the microneedles have a diameter ranging from 0.25 to 250 µm and have a height ranging from 5 to 200 µm. The functional member may have a microhole on the surface between the microneedles. The microhole may have an inner diameter corresponding to the diameter of the microneedles. The microneedles may have a bent shape.

## Description

### Field

The present invention relates to a functional member and a method of fabricating the functional member. Background

Studies have been made on functional members including a plurality of microneedles that are tiny needle-like protrusions formed on a surface of the functional members for use in various applications making use of, for example, the water repellent properties on the surface and for use in medical nanopatches. A method of fabricating microneedles is disclosed (Patent Literature 1), in which ablation processing is performed on a glass substrate by irradiating the glass substrate with, for example, a femtosecond laser beam to form a master mold having a plurality of needle-like depressions, and the patterns of the needle-like depressions are transferred to a resin to form a secondary mold having needle-like protrusions, and then a tertiary mold is formed to finally fabricate needles made of resin.

### Citation List

### Patent Literature

Patent Literature 1: Japanese Laid-open Patent Publication No. 2008-246492

### Summary

### Technical Problem

The method disclosed in Patent Literature 1 implements femtosecond laser ablation, and thus, the size of the needle-like depressions and the resulting needles is restricted to the spot size of the femtosecond laser beam. In addition, molds are formed in a plurality of processes before a final product is produced, thereby complicating the manufacturing process.

The present invention has been made in view of the problems above, and it is an object of the present invention to provide a functional member including a plurality of microneedles on a surface with more freedom of size, and a method of fabricating the functional member in an easier way.

### Solution to Problem

A functional member according to an embodiment includes: a plurality of microneedles on a surface. Further, the microneedles adjacent to each other are spaced apart by a pitch ranging from 1 to 500 µm, and the microneedles have a diameter ranging from 0.25 to 250 µm and have a height ranging from 5 to 200 µm.

In the functional member according to an embodiment, a microhole is formed on the surface between the microneedles.

In the functional member according to an embodiment, the microhole has an inner diameter corresponding to the diameter of the microneedles.

In the functional member according to an embodiment, the microneedles have a bent shape.

In the functional member according to an embodiment, the microneedles have an outer shape of a substantial upright cone with a base on the surface, the substantial upright cone having a dent on a part of an outer circumferential surface.

In the functional member according to an embodiment, the microneedles have a tip having minute indentations and protrusions having a diameter equal to or smaller than 500 nm.

In the functional member according to an embodiment, the functional member is made of stainless steel or aluminum metal, or made of resin.

In the functional member according to an embodiment, the functional member is functioned as a junction member or a catalyst retaining member.

A method of fabricating a functional member made of metal and including a plurality of microneedles on a surface according to an embodiment, the method includes: emitting a laser beam on a surface of a raw member and performing scanning with the laser beam in a first direction at a pitch d. Further, the microneedles are spaced apart by a pitch ranging from 1 to 500 µm, and the microneedles have a diameter ranging from 0.25 to 250 µm and have a height ranging from 5 to 200 µm, and a spot size radius of the laser beam is indicated as ω, and a parameter Δ relative to ω is set to satisfy 0 < Δ < 2ω, and the pitch d is set to satisfy d < 2ω + Δ.

In the method of fabricating the functional member according to according to an embodiment, the laser beam is a subnanosecond to nanosecond pulsed laser beam.

In the method of fabricating the functional member according to according to an embodiment, the surface is scanned with the laser beam only in the first direction.

In the method of fabricating a functional member according to according to an embodiment, the laser beam is used for scanning only in the first direction at a scanning speed equal to or lower than 20 mm/s.

In the method of fabricating the functional member according to according to an embodiment, when the laser beam is split into N sub-laser beams spaced apart at a pitch D, where N is an integer equal to or larger than two, and emitted to the surface, and when a certain sub-laser beam of the N sub-laser beams is used for scanning at a scan pitch I, I = N * D or I = 1/N * D is satisfied.

In the method of fabricating the functional member according to according to an embodiment, the raw member is irradiated with the laser beam and processed in an inert gas atmosphere.

In the method of fabricating the functional member according to according to an embodiment, the diameter of the microneedles is adjusted by adjusting at least one of the pitch d, the spot size radius ω, the parameter Δ, a fluence of the laser beam or the sub-laser beams, a scanning speed, and a processing atmospheric gas.

In a method of fabricating a functional member according to an embodiment made of resin and including a plurality of microneedles on a surface, the method includes: preparing a functional member fabricated by the method according to any one of claims 9 to 15 as a mold; and transferring the microneedles onto a resin. Advantageous Effects of Invention

According to the present invention, a functional member including a plurality of microneedles on a surface with more freedom of size can be achieved more easily. Brief Description of Drawings

FIG. 1 is a schematic diagram of a functional member according to an embodiment.
FIG. 2 is a diagram illustrating a microphotograph of a surface of the functional member illustrated in FIG. 1.
FIG. 3 is an enlarged view of tips of microneedles illustrated in FIG. 2.
FIG. 4 is a schematic sectional view of a surface and the vicinity of the surface of the functional member illustrated in FIG. 1.
FIG. 5 is a diagram illustrating an example method of fabricating the functional member illustrated in FIG. 1.
FIG. 6 is a diagram illustrating how to form microneedles.
FIG. 7 is a diagram illustrating an example scanning pattern of sub-laser beams.
FIG. 8 is a diagram illustrating another example scanning pattern of the sub-laser beams.
FIG. 9 is a diagram illustrating an example method of fabricating a functional member made of resin. Description of Embodiment

The following describes an embodiment of the present invention with reference to the accompanying drawings. The embodiment is not intended to limit the scope of the present invention. Same reference signs are given to the same or corresponding elements in the drawings. The drawings are illustrated schematically and thus it should be noted that, for example, the relation between dimensions of the elements and the ratio of the elements may differ from the actual ones. Dimensional relation or ratio of elements may differ between the drawings.

### (Embodiment)

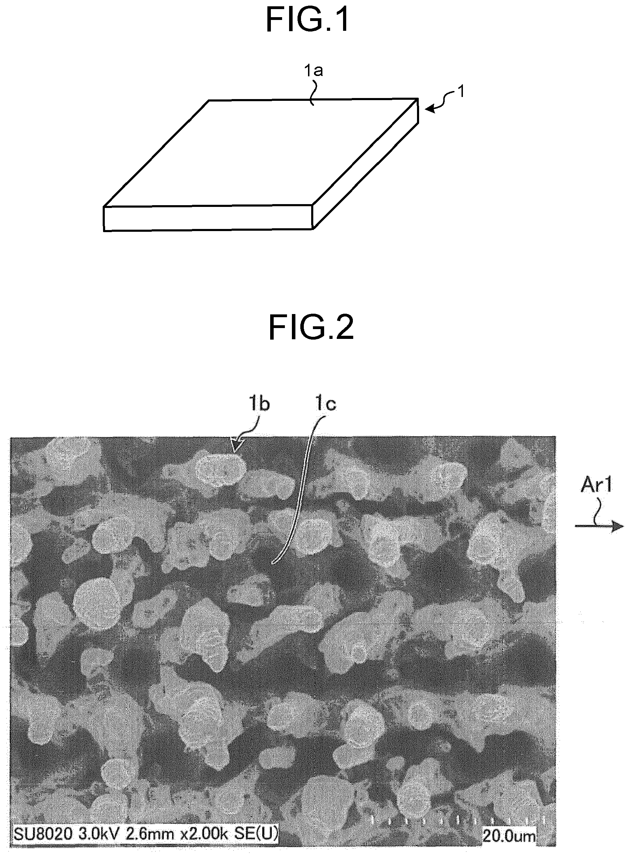

FIG. 1 is a schematic diagram of a functional member according to an embodiment. This functional member 1 is made of metal. In the present embodiment, the functional member 1 is made of stainless steel, but the functional member 1 may be made of other types of metal such as aluminum metal. The shape of the functional member 1 is, but not limited to, a plate shape in the present embodiment.

A plurality of microneedles are formed on a surface 1a of the functional member 1. FIG. 2 is a diagram illustrating a microphotograph of the surface 1a of the functional member 1. FIG. 2 includes a scale for indicating the size, and the value of one division of the scale is 2.0 µm. As illustrated in FIG. 2, the surface 1a has a plurality of microneedles 1b. The microneedles 1b are arranged in a plurality of rows in the direction indicated by an arrow Ar1.

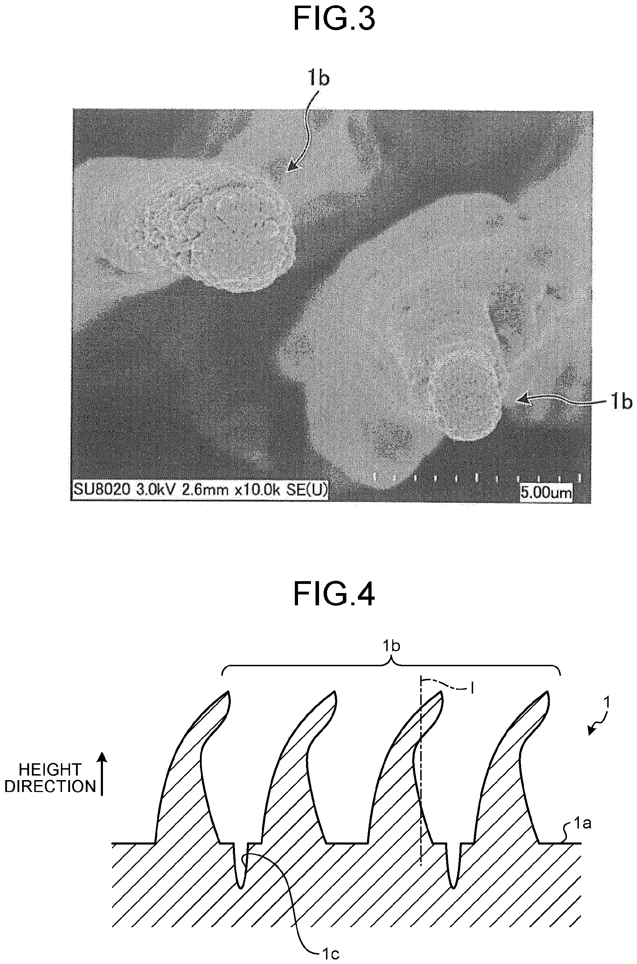

FIG. 3 is an enlarged view of tips of the microneedles 1b. FIG. 3 includes a scale for indicating the size, and the value of one division of the scale is 0.5 µm. As illustrated in FIG. 3, the tips of the microneedles 1b have minute indentations and protrusions having a diameter equal to or smaller than 500 nm.

FIG. 4 is a schematic sectional view of the surface 1a and the vicinity of the surface 1a of the functional member 1. The microneedles 1b extend in a height direction from the surface 1a. Adjacent microneedles 1b are spaced apart by a pitch ranging from 1 to 500 µm. The pitch between the adjacent microneedles 1b is a pitch between the tips of the adjacent microneedles 1b. The microneedles 1b each have a diameter ranging from 0.25 to 250 µm. The diameter of the microneedles 1b is a mean value of the diameters from a diameter close to the surface 1a to a diameter at the tip of the microneedles 1b. When the microneedles 1b have a cross-section other than a circle, the diameter of a circle having the same area of this cross-section is used as the diameter of the cross-section. The microneedles 1b have a height ranging from 5 to 200 µm from the surface 1a.

The microneedles 1b have a bent shape. For example, the microneedles 1b extend from the surface 1a such that the microneedles 1b cross an imaginary straight-line 1 perpendicular to the surface 1a and extend without crossing the straight-line 1 and then cross the straight-line 1 again. The outer shape of the microneedles 1b is, for example, a substantial upright cone with a base on the surface 1a and having a dent on a part of the outer circumferential surface. Examples of the cone include a right circular cone and an oblique circular cone.

A microhole 1c is formed on the surface 1a between the microneedles 1b (see FIG. 2). The microhole 1c has an inner diameter corresponding to, for example, the diameter of the microneedles 1b. For example, the microhole 1c is formed at substantially the middle between two adjacent microneedles 1b. The inner diameter of the microhole 1c is an inner diameter of the microhole 1c at the edge of the surface 1a.

The functional member 1 includes a plurality of microneedles 1b with much freedom of size, and the microneedles 1b having various sizes are formed on the surface 1a. This structure provides a higher water repellency. When the functional member 1 is used as a junction member having a function of contacting and joining, for example, resin, the microneedles 1b provide increased anchoring properties and increased adhesiveness, thereby increasing bonding properties. When the functional member 1 is used as a catalyst retention member, the microneedles 1b provide increased catalyst absorption properties, thereby increasing catalyst retainability. In particular, since the functional member 1 has bent microneedles 1b, such microneedles 1b further increase water repellency, bonding properties, and catalyst retainability.

### (Fabrication Method)

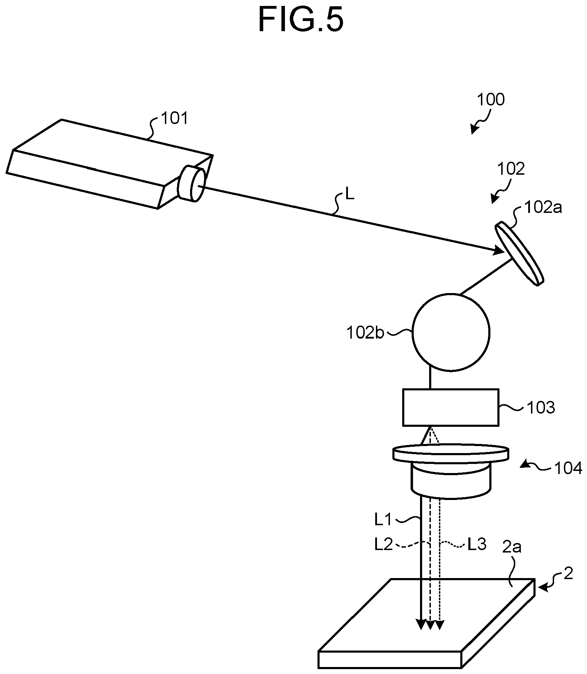

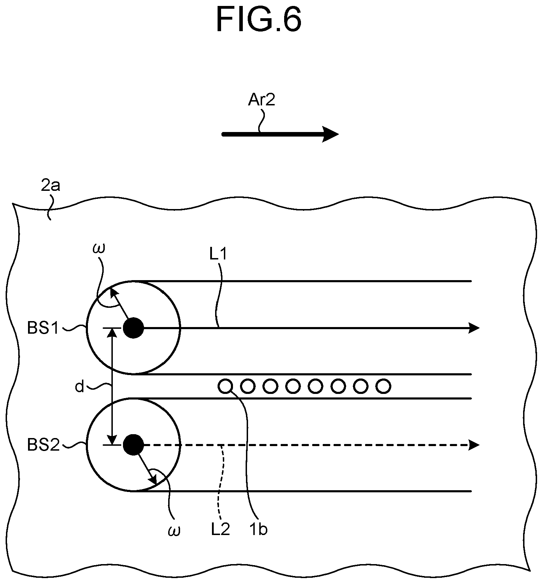

Described next is an example method of fabricating the functional member 1 with reference to FIGS. 5 and 6. A fabrication apparatus 100 for implementing this fabrication method includes a laser device 101, a galvanometer scanner 102, a diffractive optical element 103, and an fθ lens 104. A raw member 2 is a member to be processed to fabricate the functional member 1, and is a plate member made of stainless steel.

The laser device 101 includes a laser light source such as an optical fiber laser and emits, for example, a laser beam L that is a subnanosecond to nanosecond pulsed laser beam having a pulse width ranging from 0.1 to 999 nanoseconds. Using the subnanosecond to nanosecond pulsed laser beam can increase the scanning speed (processing speed) compared to using a femtosecond pulsed laser beam. This configuration can increase manufacturability of the functional member 1.

The galvanometer scanner 102 includes galvanometer mirrors 102a, 102b each rotated by an electric motor. The laser beam L emitted by the laser device 101 is reflected from the rotating galvanometer mirrors 102a, 102b to use the laser beam L for scanning.

The diffractive optical element 103 splits the laser beam L into N sub-laser beams, where N is an integer equal to or larger than two. In this fabrication method, the diffractive optical element 103 splits the laser beam L into three sub-laser beams L1, L2, L3.

The fθ lens 104 focuses the laser beam, which is used for equiangular scanning by the galvanometer scanner 102, on a certain plane and scans the plane at an equal speed. The certain plane is set to be coincident with a surface 2a of the raw member 2 placed on a stage (not illustrated). Thus, the sub-laser beams L1, L2, L3 are emitted for scanning while being focused on the surface 2a of the raw member 2 by the fθ lens 104. Emitting the sub-laser beams L1, L2, L3 to the surface 2a of the raw member 2 can form the microneedles 1b and the inner diameter of the microholes 1c.

In forming the microneedles 1b and the microholes 1c, it is preferred that the raw member 2, which is a processing target, is irradiated with the laser beam L and processed in an inert gas atmosphere such as nitrogen or argon. This configuration prevents acceleration of process due to oxygen and can stably form the microneedles 1b and the microholes 1c.

FIG. 6 is a diagram illustrating how to form the microneedles 1b. As illustrated in FIG. 6, the sub-laser beams L1, L2 are emitted to the surface 2a of the raw member 2, and the galvanometer scanner 102 performs scanning with the sub-laser beams L1, L2 in a first direction (indicated by an arrow Ar2) with a pitch d therebetween. The sub-laser beam L3 is used for scanning in the first direction with the pitch d from the sub-laser beam L2, but for ease of description, the sub-laser beam L3 is omitted from the illustration and from the description as appropriate.

Let the spot size radius of respective beam spots BS1, BS2 of the sub-laser beams L1, L2 on the surface 2a be ω. In this case, a parameter Δ relative to ω is set to satisfy 0 < Δ < 2ω, and the pitch d is set to satisfy d < 2ω + Δ. Scanning the raw member 2 with the sub-laser beams L1, L2 melts and removes the material of the raw member 2 in certain ranges from the respective centers of the beam spots BS1, BS2 of the sub-laser beams L1, L2, but the material of the raw member 2 in a region between the sub-laser beams L1, L2 remains and becomes a row of microneedles 1b. In the same manner, another row of microneedles 1b is formed in a region between the sub-laser beams L2, L3. The row of microneedles 1b formed by the sub-laser beams L1, L2 and the row of microneedles 1b formed by the sub-laser beams L2, L3 are spaced apart at about the pitch d.

The diameter of the microneedles 1b can be adjusted by adjusting at least one of the pitch d, the spot size radius ω. Δ, the fluence of the laser beam L or the sub-laser beams L1, L2, L3, the scanning speed, and the processing atmospheric gas. It is desirable that Δ is set appropriately in forming the rows of microneedles 1b. It is preferred that the scanning speed has a certain low value such as 20 m/s or lower. The pitch d is, for example, 10 µm, and the spot size radius ω is, for example, 10 µm. In particular, it is preferred that the diameter of the microneedles 1b is adjusted by adjusting the pitch d and the spot size radius ω.

In the first scan, the sub-laser beams L1, L2, L3 are used for scanning in the first direction from a first end of a range to be formed with the microneedles 1b to a second end opposite to the first end, at which the first scan ends and then the process proceeds to the second scan. In the second scan, the sub-laser beams L1, L2, L3 are used for scanning from an irradiation start position at the first end. This irradiation start position is shifted from the irradiation position of the first scan in a direction orthogonal to the first direction, and the sub-laser beams L1, L2, L3 are used for scanning in the first direction. In the third scan and after, the sub-laser beams L1, L2, L3 are used for scanning only in the first direction in the same manner as described above, and many microneedles 1b are formed in a desirable range. The functional member 1 can be fabricated in this manner.

The process of splitting the laser beam L into the sub-laser beams L1, L2, L3 and performing scanning with the sub-laser beams to form the microneedles 1b can reduce the takt time to about one-third compared to the takt time of a case using the laser beam L to form the microneedles 1b.

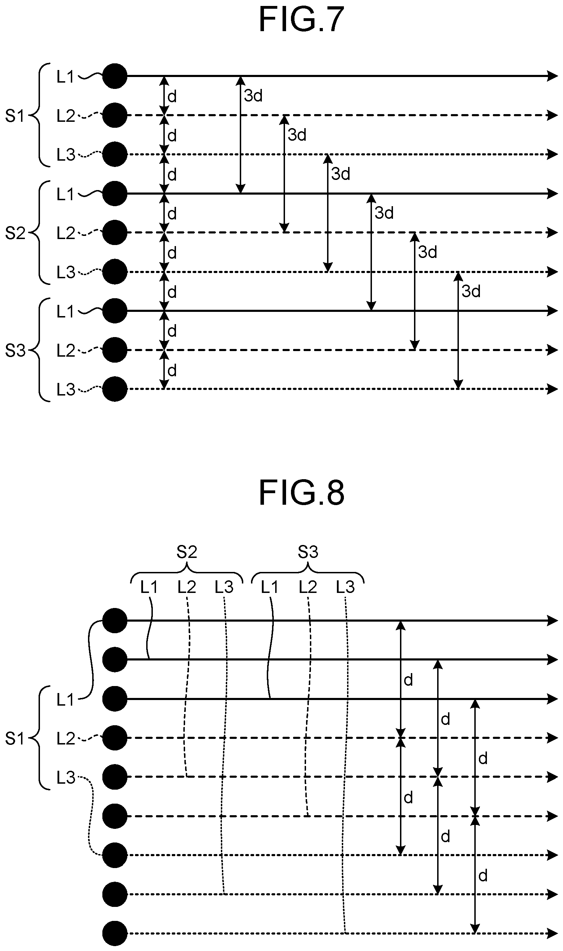

FIG. 7 is a diagram illustrating an example scanning pattern of the sub-laser beams L1, L2, L3. FIG. 7 illustrates a scanning pattern seen in a direction perpendicular to the surface 2a of the raw member 2. When the number of sub-laser beams is N and the respective N sub-laser beams are spaced apart at a pitch D, and when a certain sub-laser beam of the N sub-laser beams is used for scanning at a scan pitch I, the scanning pattern satisfies I = N * D.

Specifically, N is three in FIG. 7. In a first scan S1, the sub-laser beams L1, L2, L3 are used for scanning while being spaced apart at a pitch D = d. In a subsequent second scan S2, the sub-laser beam L1 in the second scan is used for scanning at a scan pitch I = 3d from the sub-laser beam L1 in the first scan. In the same manner, in a third scan S3, the sub-laser beam L1 in the third scan is used for scanning at the scan pitch I = 3d from the sub-laser beam L1 in the second scan. The sub-laser beams L2, L3 are also used for scanning at the scan pitch I = 3d. In this regard, the sub-laser beams L1, L2, L3 are used for scanning at the equal pitch d.

FIG. 8 is a diagram illustrating another example scanning pattern of the sub-laser beams L1, L2, L3. FIG. 8 illustrates a scanning pattern seen in a direction perpendicular to the surface 2a of the raw member 2. When the number of sub-laser beams is N and the respective N sub-laser beams are spaced apart at a pitch D, and when a certain sub-laser beam of the N sub-laser beams is used for scanning at a scan pitch I, the scanning pattern satisfies I = 1/N * D.

Specifically, N is three in FIG. 8. In the first scan S1, the sub-laser beams L1, L2, L3 are used for scanning while being spaced apart at a pitch D = d. In the subsequent second scan S2, the sub-laser beam L1 in the second scan is used for scanning at a scan pitch I = 1/3 * d from the sub-laser beam L1 in the first scan. In the same manner, in the third scan S3, the sub-laser beam L1 in the third scan is used for scanning at the scan pitch I = 1/3 * d from the sub-laser beam L1 in the second scan. The sub-laser beams L2, L3 are also used for scanning at the scan pitch I = 1/3 * d. In this regard, the sub-laser beams L1, L2, L3 are used for scanning at the equal pitch of I = 1/3 * d. The pitch I in this case corresponds to the pitch d.

N is three in FIGS. 7 and 8. When N is an integer equal to or larger than two and I = N * D or I = 1/N * D is satisfied, N sub-laser beams can be used for scanning at an equal pitch. When the laser beam L is not split into sub-laser beams, N is set to one and both I = N * D and I = 1/N * D are satisfied. In this case, the laser beam L is used for scanning at a pitch D or I. When N is equal to or larger than two, the takt time can be reduced to, for example, about one-Nth.

Using the functional member 1 fabricated by the fabrication method described above as a mold and transferring the microneedles 1b onto resin can fabricate a resin functional member including a plurality of microneedles on a surface.

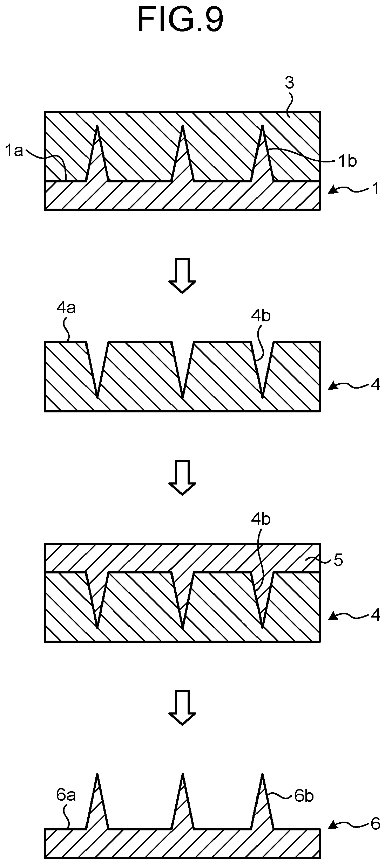

FIG. 9 is a diagram illustrating an example method of fabricating a functional member made of resin. First, the functional member 1 including a plurality of microneedles 1b on the surface 1a is prepared as a mold and a resin 3 such as thermosetting resin is applied to the surface 1a, and the resin 3 is cured and then removed from the functional member 1. In this process, a functional member 4 made of resin having a plurality of microholes 4b on a surface 4a is fabricated. The microholes 4b have a shape transferred from the shape of the microneedles 1b.

Subsequently, the functional member 4 is used as a mold and a resin 5 such as thermosetting resin is applied to the surface 4a, and the resin 5 is cured and then removed from the functional member 4. In this process, a functional member 6 made of resin including a plurality of microneedles 6b on a surface 6a is fabricated. The microholes 6b have a shape transferred from the shape of the microneedles 1b of the functional member 1.

The embodiment above is not intended to limit the scope of the present invention. Combinations, as appropriate, of the constituent elements described above are also included in the scope of the present invention. Further effects and modifications can be easily derived by those skilled in the art. Accordingly, broader embodiments of the present invention are not limited to the embodiment above and various modifications may be made.

### Industrial Applicability

As described above, the functional member and the method of fabricating the functional member according to the present invention are suitable for use in various applications using, for example, water repellent properties of the functional member and for use in medical nanopatches.

### Reference Signs List

1, 4, 6 Functional member
1a, 2a, 4a, 6a Surface
1b, 6b Microneedle
1c, 4b Microhole
2 Raw member
100 Fabrication apparatus
101 Laser device
102 Galvanometer scanner
102a, 102b Galvanometer mirror
103 Diffractive optical element
104 fθ lens
Ar1, Ar2 Arrow
BS1, BS2 Beam spot
L Laser beam
L1, L2, L3 Sub-laser beam
S1, S2, S3 Scan

## Claims

1. A functional member comprising:
a plurality of microneedles on a surface, wherein
the microneedles adjacent to each other are spaced apart by a pitch ranging from 1 to 500 µm, and the microneedles have a diameter ranging from 0.25 to 250 µm and have a height ranging from 5 to 200 µm.

2. The functional member according to claim 1, wherein a microhole is formed on the surface between the microneedles.

3. The functional member according to claim 2, wherein the microhole has an inner diameter corresponding to the diameter of the microneedles.

4. The functional member according to any one of claims 1 to 3, wherein the microneedles have a bent shape.

5. The functional member according to any one of claims 1 to 3, wherein the microneedles have an outer shape of a substantial upright cone with a base on the surface, the substantial upright cone having a dent on a part of an outer circumferential surface.

6. The functional member according to any one of claims 1 to 5, wherein the microneedles have a tip having minute indentations and protrusions having a diameter equal to or smaller than 500 nm.

7. The functional member according to any one of claims 1 to 6, wherein the functional member is made of stainless steel or aluminum metal, or made of resin.

8. The functional member according to any one of claims 1 to 7, wherein the functional member is functioned as a junction member or a catalyst retaining member.

9. A method of fabricating a functional member made of metal and including a plurality of microneedles on a surface, the method comprising:
emitting a laser beam on a surface of a raw member and performing scanning with the laser beam in a first direction at a pitch d, wherein
the microneedles are spaced apart by a pitch ranging from 1 to 500 µm, and the microneedles have a diameter ranging from 0.25 to 250 µm and have a height ranging from 5 to 200 µm, and
let a spot size radius of the laser beam be ω, and a parameter Δ relative to ω is set to satisfy 0 < Δ < 2ω, and the pitch d is set to satisfy d < 2ω + Δ.

10. The method of fabricating a functional member according to claim 9, wherein the laser beam is a subnanosecond to nanosecond pulsed laser beam.

11. The method of fabricating a functional member according to claim 9 or 10, wherein the surface is scanned with the laser beam only in the first direction.

12. The method of fabricating a functional member according to any one of claims 9 to 11, wherein the laser beam is used for scanning only in the first direction at a scanning speed equal to or lower than 20 mm/s.

13. The method of fabricating a functional member according to any one of claims 9 to 12, wherein
when the laser beam is split into N sub-laser beams spaced apart at a pitch D, where N is an integer equal to or larger than two, and emitted to the surface, and when a certain sub-laser beam of the N sub-laser beams is used for scanning at a scan pitch I, I = N ^{∗} D or I = 1/N ^{∗} D is satisfied.

14. The method of fabricating a functional member according to any one of claims 9 to 13, wherein the raw member is irradiated with the laser beam and processed in an inert gas atmosphere.

15. The method of fabricating a functional member according to any one of claims 9 to 14, wherein the diameter of the microneedles is adjusted by adjusting at least one of the pitch d, the spot size radius ω, the parameter Δ, a fluence of the laser beam or the sub-laser beams, a scanning speed, and a processing atmospheric gas.

16. A method of fabricating a functional member made of resin and including a plurality of microneedles on a surface, the method comprising:
preparing a functional member fabricated by the method according to any one of claims 9 to 15 as a mold; and
transferring the microneedles onto a resin.
